# EUROPEAN PATENT APPLICATION

(11) **EP 2 003 206 A1**
(43) Date of publication of application: **17.12.2008**
(21) Application number: 08163102.0
(22) Date of filing: 12.09.2005
(51) Int. Cl.: C12N 15/82

(54) **Promoter molecules for use in plants**

(30) Priority: 14.09.2004 US 609770 P
(62) Divisional of application: 05796642.6
(71) Applicant: Monsanto Technology, LLC, St. Louis, MO 63167 (US)
(72) Inventor: Ruezinsky, Diane, M., St. Louis, MO 63167 (US); Tzafrir, Iris, St. Louis, MO 63167 (US); Conner, Timothy, W., St. Louis, MO 63167 (US)
(74) Representative: Helbing, Jörg

(57) **Abstract**

The present invention relates to polynucleotide molecules for regulating gene expression in plants. In particular, the invention relates to promoters isolated from *Glycine max* and *Arabidopsis thaliana* that are useful for regulating gene expression of heterologous polynucleotide molecules in plants. The invention also relates to expression constructs and transgenic plants containing the heterologous polynucleotide molecule.

SEQUENCE LISTING

<110>  Monsanto Technology LLC

<120>  Promoter Molecules for Use in Plants

<130>  MONS:055US

<150>  PCT/US2005/032496
<151>  2005-09-12

<150>  US 60/609,770
<151>  2004-09-14

<160>  5

<170>  PatentIn version 3.3

<210>  1
<211>  663
<212>  DNA
<213>  Glycine max

<400>  1
cgtaaacctt tttatataag ttttatccaa ctgtagtaca gatgatttag ttcactaaaa      60

ttgtttatat accttttact caagttacca ataattttct ttcagtatta tcgagagtca     120

agttagtacc gaaaaaccta ttgtattatt cttgagttac agtgcgctag gctcagcatt     180

actgagagct ttttcgatgt catcgatata aaatttaacg agatggtgga gtatttattg     240

atgaatatta agtcatcaat atacaatgag tttttgctat tacaactcag taaatctttt     300

gtgcaattaa ttattttgct ttcaattgat cccttgtgta cgttacattg atgctaatgg     360

atgaaattag ccaacagaac tcttaatatg aaaaatgaga gtatctggtc tgaaattata     420

catttttttt accatcacaa ttgaacataa aagaaggact atcacaattg ataaggatat     480

tagcaattaa ggaaatagat tccttatatt agcttccacc tcaaaactga attttgttgt     540

aattaaggaa aaaatgctga catccataat tatgagtatc tcataacgtg acaaaaaaag     600

ttaaatatct gtattttgga taaatatttt ttaagcttta ttttatttta tctgagggtc     660

ata                                                                   663


<210>  2
<211>  669
<212>  DNA
<213>  Glycine max

<400>  2
ggtatccata tcaagttctc gagtagttcc ccaccctcat cttcaattcc gcatgagctc      60

tagctctagc aacctaaatc taattaaagt ttgaaagatt atgtagagcc aaactttaag     120

tgtcaatttt cataatgagt taaaccttaa atgtcaattt tcatcacagg gtaataggat     180

tgagtgctta ggggactaac ttacaatgag gtgtcagttc gtaaatacgt gtaatgtagt     240

ctctgtcgcc aacatgtgcc acgagaaatc agtaatggtt aacctgatgt caccatggtg 300

ctcctttgca ctagaagaca tggatgcagc agaagagtgt gataagagca tgtcgaattt 360

caacagtgat ggctttgtgt tttgtttgtt cttactttag tttattttaa tcttttttta 420

ccgttctggg gcaccaagtc aaaagagttt agtcaagcac aatcgcacaa ggctctccct 480

ctctctcccc ctgtcccaca agaaagtgaa ataacaaaca cattgtctcc ttagattcct 540

ttcttttcta tttgaagcgc actctatccc agtctctctc tgatttccta tatcagctgg 600

tgggacactg gttatgtttc ttttgtcagg attctcttgc tctcactata tcaaggagga 660

gaggggaga 669

<210> 3
<211> 34
<212> DNA
<213> Artificial

<220>
<223> Synthetic primer

<400> 3
gcccgggcgg tttaccggaa ttgtatgtca atgg 34

<210> 4
<211> 29
<212> DNA
<213> Artificial

<220>
<223> Synthetic primer

<400> 4
tcccatggtc acttttctct ctcttgtgc 29

<210> 5
<211> 964
<212> DNA
<213> Arabidopsis thaliana

<400> 5
ggtttaccgg aattgtatgt caatggtgtt aaccacatga cgtcctatac ttcatatatg 60

tggtcatgta atacatctac ttcgtgtcta cttcgtgtag ctggatatac aatgtatagt 120

aggtatgtgt gaccatgtat tctcttatac tttgtttacc tagcaatctt ttttttaaat 180

taaaataaat atgcggttta gatatgaaac tacccaacaa atttaacatt ttaaacgttc 240

ataacgtaaa acgacgtcgt tatagacaca tattttccat gtgtctgctg acttatcatc 300

ttcacggagt tgactaacac ccgttacttt gactctgaat tttgtacttt ttcttaagtt 360

gaggtatgaa attcaaataa atatgcggtt aatatatgaa aatacccaac aaattttttt 420

ggatacgaaa atacactcag aaaatagtac gggtatgaaa ataccctttt cccgtatttg 480

atacatgtct aattcggttc aaataaaccg aatatgaaaa ttttcagttt tatttcggaa 540

gttaaataaa tctagataac cgacctgaaa aacccgagtc ccgaccgaac cgaaccgaaa 600

ttaaattcgg tttaattcgg aagcatttcc aaaaaccgaa attccctaaa accgaataac 660

ccgacccgat taaaccgatt tgccgaactc ccaggcctaa attcacactt ggcttagaaa 720

aactctttgt agatgttaaa attcggtaaa attaacctca ccaaagctaa ttattaccag 780

gtgaagaaag cattaaaatt tcaaagtgtg tatgacagag gttttagaaa gcgactgatg 840

tacggacata tcaacaactc ccctataaag atactcagct aaacacaaaa acagaatcta 900

ttctcaacac aacactaaag acaattgtac caaccacaca accacaagag agagaaaagt 960

gacc 964

## Description

### BACKGROUND OF THE INVENTION

This application claims the priority of U.S. Provisional Application No. 60/609,770, filed September 14, 2004, the disclosure of which is incorporated herein by reference in its entirety.

### FIELD OF THE INVENTION

The invention relates to the field of molecular biology and more specifically to polynucleotide molecules useful for the expression of transgenes in plants. The invention relates to the P-Gm.701202739 and P-Gm.701209813 promoters isolated from *Glycine max* and to the P-At.TT2 promoter isolated from *Arabidopsis thaliana.* The promoters are useful for expression of transgenes of agronomic importance in crop plants.

### BACKGROUND OF THE INVENTION

One of the goals of plant genetic engineering is to produce plants with agronomically desirable characteristics or traits. Advances in genetic engineering have provided the requisite tools to transform plants to contain and express foreign genes. The technological advances in plant transformation and regeneration have enabled researchers to take an exogenous polynucleotide molecule, such as a gene from a heterologous or native source, and incorporate that polynucleotide molecule into a plant genome. The gene can then be expressed in a plant cell to exhibit the added characteristic or trait. In one approach, expression of a gene in a plant cell or a plant tissue that does not normally express such a gene may confer a desirable phenotypic effect. In another approach, transcription of a gene or part of a gene in an antisense orientation may produce a desirable effect by preventing or inhibiting expression of an endogenous gene.

Promoters are polynucleotide molecules that comprise the 5' regulatory elements which play an integral part in the overall expression of genes in living cells. Isolated promoters that function in plants are useful for modifying plant phenotypes through the methods of genetic engineering. The first step in the process to produce a transgenic plant includes the assembly of various genetic elements into a polynucleotide construct. The construct includes a transcribable polynucleotide molecule (gene of interest) that confers a desirable phenotype when expressed (transcribed) in the plant cells by a promoter that is operably linked to the gene of interest. A promoter in a construct may be homologous or heterologous to the gene of interest also contained therein. The construct is then introduced into a plant cell by various methods of plant transformation to produce a transformed plant cell and the transformed plant cell is regenerated into a transgenic plant. The promoter controls expression of the gene of interest to which the promoter is operably linked and thus affects the characteristic or trait conferred by the expression of the transgene in plants.

For production of transgenic plants with various desired characteristics, it would be advantageous to have a variety of promoters to provide gene expression such that a gene is transcribed efficiently in the amount necessary to produce the desired effect. The commercial development of genetically improved germplasm has also advanced to the stage of introducing multiple traits into crop plants, often referred to as a gene stacking approach. In this approach, multiple genes conferring different characteristics of interest can be introduced into a plant. It is often desired when introducing multiple genes into a plant that each gene is modulated or controlled for optimal expression, leading to a requirement for diverse regulatory elements. In light of these and other considerations, it is apparent that optimal control of gene expression and regulatory element diversity are important in plant biotechnology.

A variety of different types or classes of promoters can be used for plant genetic engineering. Promoters can be classified on the basis of characteristics such as temporal or developmental range, levels of transgene expression, or tissue specificity. For example, promoters referred to as constitutive promoters are capable of transcribing operably linked genes efficiently and expressing those genes in multiple tissues. Different types of promoters can be obtained by isolating the upstream 5' regulatory regions of genes that are transcribed and expressed in the desired manner, e.g., constitutive, tissue enhanced, or developmentally induced.

Numerous promoters, which are active in plant cells, have been described in the literature. These include the nopaline synthase (nos) promoter and octopine synthase (ocs) promoters carried on tumor-inducing plasmids of *Agrobacterium tumefaciens* and the caulimovirus promoters such as the Cauliflower Mosaic Virus (CaMV) 19S or 35S promoter (U.S. Patent 5,352,605), CaMV 35S promoter with a duplicated enhancer (U.S. Patents 5,164,316; 5,196,525; 5,322,938; 5,359,142; and 5,424,200), and the Figwort Mosaic Virus (FMV) 35S promoter (U.S. Patent 5,378,619). These promoters and numerous others have been used in the creation of constructs for transgene expression in plants. Other useful promoters are described, for example, in U.S. Patents 5,391,725; 5,428,147; 5,447,858; 5,608,144; 5,614,399; 5,633,441; 6,232,526; and 5,633,435, all of which are incorporated herein by reference.

Promoters are also needed for expression of genes in seeds for the production of plant oils and other traits. The seed coat is a tissue of maternal origin. Once fertilization has taken place the seed coat is responsible for maintaining the integrity of the fertilized life within the seed, by both sheltering the embryo and providing adequate nutrition, until such time as conditions for growth are present. The present invention describes promoters found to express in the seed coat.

While previous work has provided a number of promoters useful to direct transcription in transgenic plants, there is still a need for novel promoters with beneficial expression characteristics. In particular, there is a need for promoters that are capable of directing expression of exogenous genes, for oil production, in seeds. Many previously identified promoters fail to provide the patterns or levels of expression required to fully realize the benefits of expression of selected seed-specific oil-associated genes in transgenic plants. There is, therefore, a need in the art of plant genetic engineering for novel promoters for use in oilseeds.

### SUMMARY OF THE INVENTION

The present invention relates to promoters that are expressed in the seed coat. Seed coat promoters are useful for production of transgenic plants with desired seed traits. These include, but are not limited to, altering nutrient uptake, carbon storage, metabolism and transport, carbon/nitrogen biosynthesis, phenylpropanoid biosynthesis, seed composition or size, oil content, protein quality, or micronutrient quality.

In one embodiment, the present invention provides a promoter comprising a polynucleotide sequence substantially homologous to a polynucleotide sequence selected from the group consisting of SEQ ID NO:1, SEQ ID NO:2 and SEQ ID NO:5, and fragments thereof that are capable of regulating transcription of operably linked polynucleotide molecules. Provided by the invention in particular embodiments are polynucleotide sequences comprising at least about 70% sequence identity to any of these sequences, including sequences with about 75%, 80%, 83%, 85%, 88%, 90%, 92%, 94%, 95%, 96%, 98%, 99% or more sequence identity to any one or more sequence of SEQ ID NO:1, SEQ ID NO:2 or SEQ ID NO:5 or a fragment thereof capable of regulating transcription of operably linked polynucleotide molecules, e.g., having promoter activity. In specific embodiments, a fragment of a sequence provided herein is defined as comprising at least about 30, 40, 50, 75, 100, 125, 150, 200, 250, 300, 350, 400, 450, 500, 600, 750, 800, or more contiguous nucleotides of any of the promoter sequences described herein such as, for example, the nucleic acid sequence of SEQ ID NO:1, SEQ ID NO:2 or SEQ ID NO:5.

In another embodiment, the invention provides a plant expression construct comprising a promoter described herein, for example, comprising a polynucleotide sequence substantially homologous to a polynucleotide sequence selected from the group consisting of SEQ ID NOs: 1, 2, and 5, or any fragments or regions thereof, wherein said promoter is operably linked to a transcribable polynucleotide molecule operably linked to a 3' transcription termination polynucleotide molecule.

In yet another embodiment, the invention provides a transgenic plant stably transformed with a plant expression construct comprising a promoter including a polynucleotide sequence substantially homologous to a polynucleotide sequence selected from the group consisting of SEQ ID NOs: 1, 2, and 5, or any fragments thereof, wherein said promoter is operably linked to a transcribable polynucleotide molecule, which can be operably linked to a 3' transcription termination polynucleotide molecule.

In another embodiment, the invention provides a method of making a vegetable oil, comprising the steps of incorporating into the genome of an oilseed plant a promoter of the present invention operably linked to a transcribable polynucleotide molecule conferring altered oil and/or protein content, growing the oilseed plant to produce oilseeds, and extracting the oil and/or protein from the oilseed.

The foregoing and other aspects of the invention will become more apparent from the following detailed description and accompanying drawings.

### BRIEF DESCRIPTION OF THE SEQUENCES

SEQ ID NO: 1 sets forth a polynucleotide sequence of a *Glycine max* P-Gm.701202739 promoter.
SEQ ID NO: 2 sets forth a polynucleotide sequence of a *Glycine max* P-Gm.701209813 promoter.
SEQ ID NOs: 3-4 set forth primer sequences.
SEQ ID NO: 5 sets forth a polynucleotide sequence of an *Arabidopsis thaliana* PAt.TT2 promoter.

### BRIEF DESCRIPTION OF THE DRAWINGS

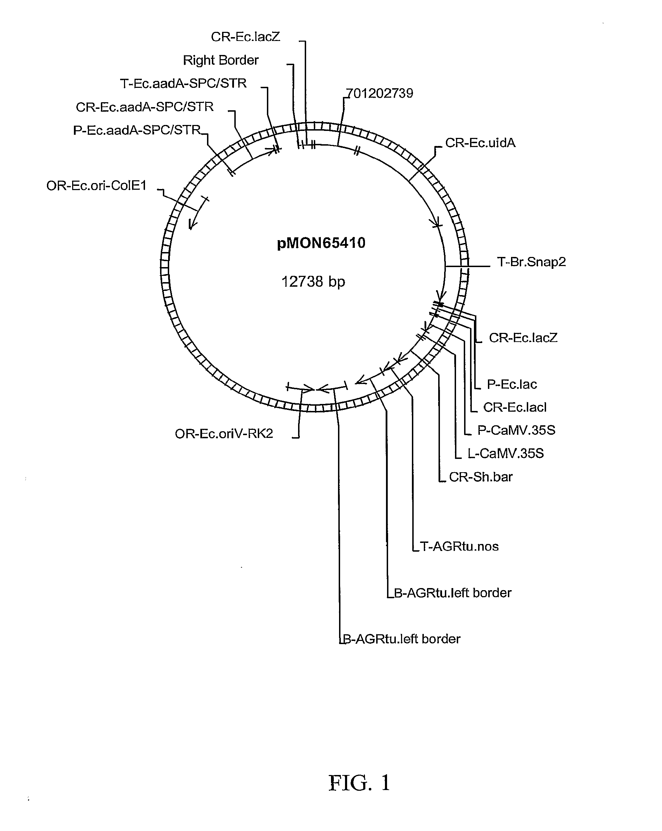

Figure 1 depicts pMON65410.

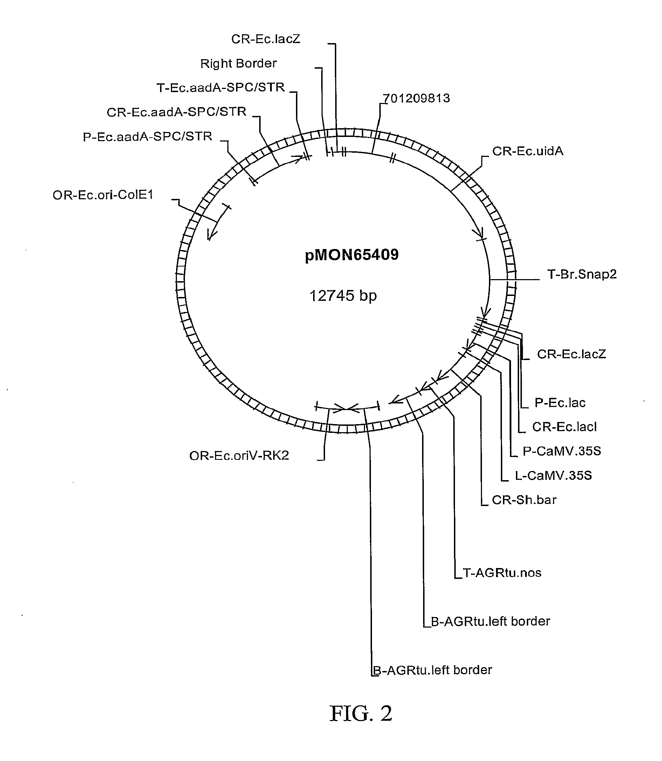

Figure 2 depicts pMON65409.

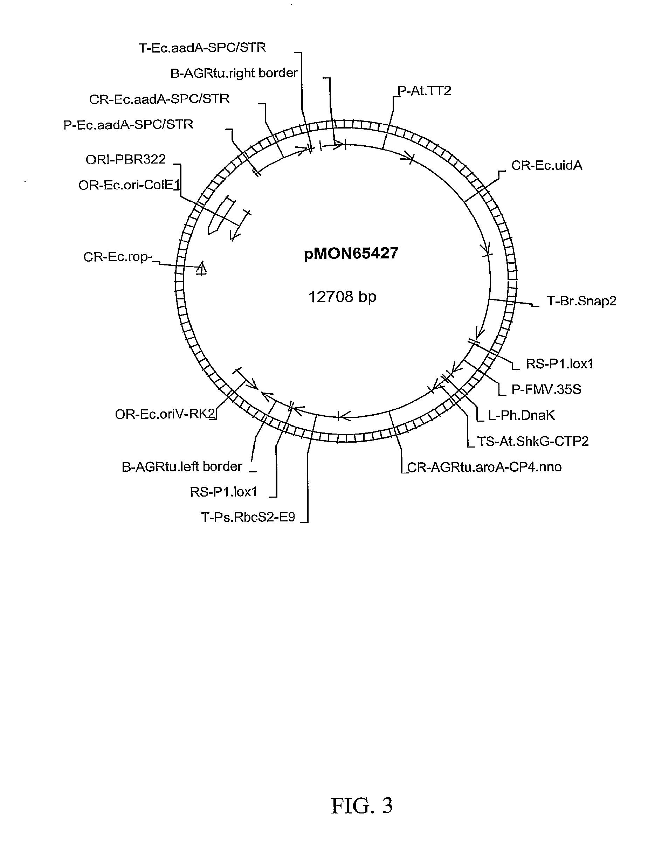

Figure 3 depicts pMON65427.

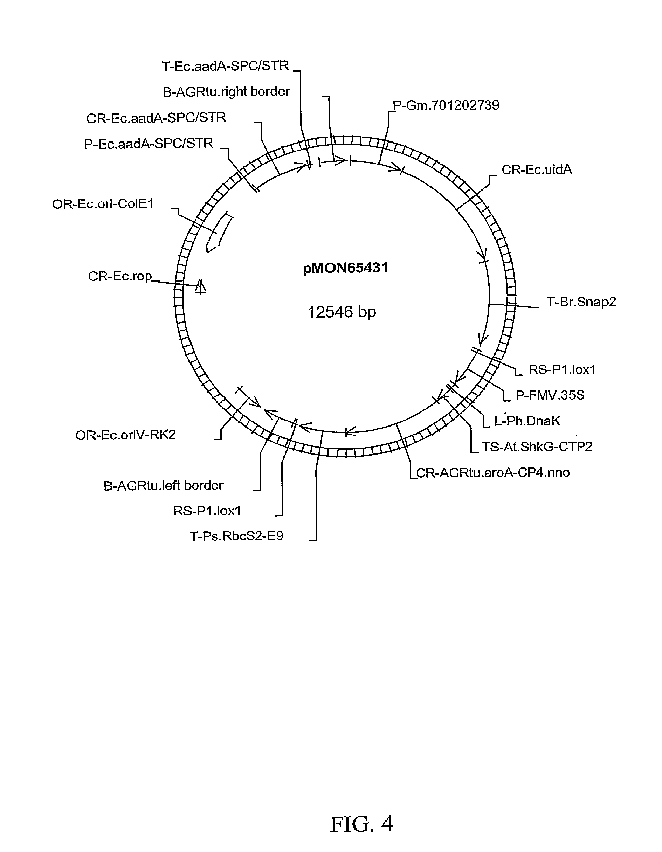

Figure 4 depicts pMON65431.

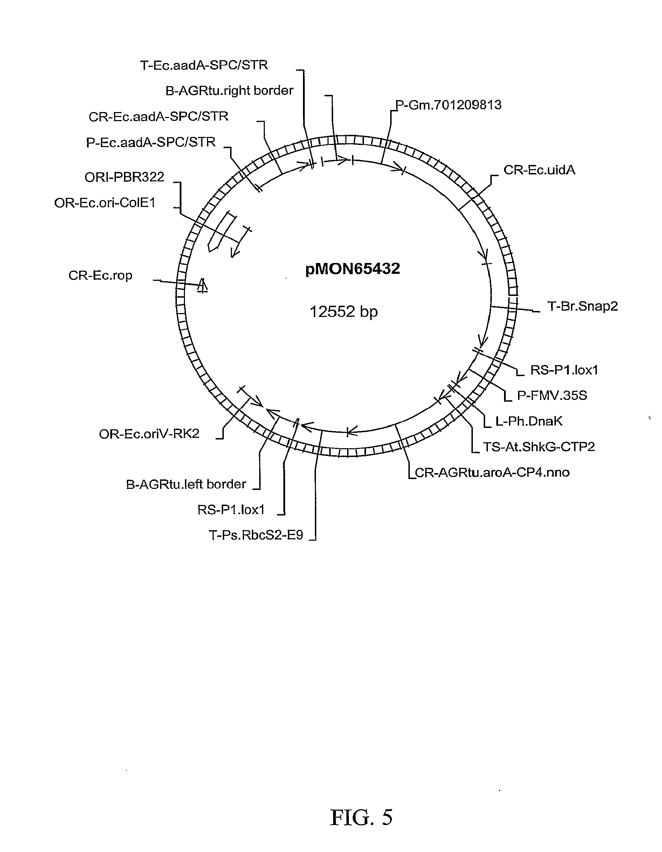

Figure 5 depicts pMON65432.

### DETAILED DESCRIPTION OF THE INVENTION

The following definitions and methods are provided to better define the present invention and to guide those of ordinary skill in the art in the practice of the present invention. Unless otherwise noted, terms are to be understood according to conventional usage by those of ordinary skill in the relevant art.

As used herein, the phrase "polynucleotide molecule" refers to the single- or double-stranded DNA or RNA of genomic or synthetic origin, *i.e.,* a polymer of deoxyribonucleotide or ribonucleotide bases, respectively, read from the 5' (upstream) end to the 3' (downstream) end.

As used herein, the phrase "polynucleotide sequence" refers to the sequence of a polynucleotide molecule. The nomenclature for DNA bases as set forth at 37 CFR § 1.822 is used.

### Promoters

As used herein, the term "promoter" refers to a polynucleotide molecule that in its native state is located upstream or 5' to a translational start codon of an open reading frame (or protein-coding region) and that is involved in recognition and binding of RNA polymerase II and other proteins (trans-acting transcription factors) to initiate transcription. A "plant promoter" is a native or non-native promoter that is functional in plant cells. Constitutive plant promoters are functional in most or all tissues of a plant throughout plant development. Any plant promoter can be used as a 5' regulatory element for modulating expression of a particular gene or genes operably associated thereto. When operably linked to a transcribable polynucleotide molecule, a promoter typically causes the transcribable polynucleotide molecule to be transcribed in a manner that is similar to that of which the promoter is normally associated. Plant promoters can include promoters produced through the manipulation of known promoters to produce artificial, chimeric, or hybrid promoters. Such promoters can also combine cis-elements from one or more promoters, for example, by adding a heterologous regulatory element to an active promoter with its own partial or complete regulatory elements. Thus, the design, construction, and use of chimeric or hybrid promoters comprising at least one cis-element of SEQ ID NOs: 1, 2, or 5 for modulating the expression of operably linked polynucleotide sequences is encompassed by the present invention.

As used herein, the term "cis-element" refers to a cis-acting transcriptional regulatory element that confers an aspect of the overall control of gene expression. A cis-element may function to bind transcription factors, trans-acting protein factors that regulate transcription. Some cis-elements bind more than one transcription factor, and transcription factors may interact with different affinities with more than one cis-element. The promoters of the present invention desirably contain cis-elements that can confer or modulate gene expression. Cis-elements can be identified by a number of techniques, including deletion analysis, *i*.*e*., deleting one or more nucleotides from the 5' end or internal to a promoter; DNA binding protein analysis using DNase I footprinting, methylation interference, electrophoresis mobility-shift *assays*, *in vivo* genomic footprinting by ligation-mediated PCR, and other conventional assays; or by DNA sequence similarity analysis with known cis-element motifs by conventional DNA sequence comparison methods. The fine structure of a cis-element can be further studied by mutagenesis (or substitution) of one or more nucleotides or by other conventional methods. Cis-elements can be obtained by chemical synthesis or by isolation from promoters that include such elements, and they can be synthesized with additional flanking nucleotides that contain useful restriction enzyme sites to facilitate subsequence manipulation.

In one embodiment, the promoters of the present invention comprise multiple cis-elements each of which confers a different aspect to the overall control of gene expression. In a preferred embodiment, cis-elements from the polynucleotide molecules of SEQ ID NOs: 1, 2, or 5 are identified using computer programs designed specifically to identify cis-element, domains, or motifs within sequences. Cis-elements may either positively or negatively regulate gene expression, depending on the conditions. The present invention therefore encompasses cis-elements of the disclosed promoters.

As used herein, the phrase "substantially homologous" refers to polynucleotide molecules that generally demonstrate a substantial percent sequence identity with the promoters provided herein. Of particular interest are polynucleotide molecules wherein the polynucleotide molecules function in plants to direct transcription and have at least about 70% sequence identity, at least about 80% sequence identity, at least about 90% sequence identity, or even greater sequence identity, such as 98% or 99% sequence identity with the polynucleotide sequences of the promoters described herein. Polynucleotide molecules that are capable of regulating transcription of operably linked transcribable polynucleotide molecules and are substantially homologous to the polynucleotide sequences of the promoters provided herein are encompassed within the scope of this invention.

As used herein, the phrase "percent sequence identity" refers to the percentage of identical nucleotides in a linear polynucleotide sequence of a reference polynucleotide molecule (or its complementary strand) as compared to a test polynucleotide molecule (or its complementary strand) when the two sequences are optimally aligned (with appropriate nucleotide insertions, deletions, or gaps totaling less than 20% of the reference sequence over the window of comparison). Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and preferably by computerized implementations of these algorithms such as GAP, BESTFIT, FASTA, and TFASTA available as part of the GCG® Wisconsin Package® (Accelrys Inc., San Diego, CA). An "identity fraction" for aligned segments of a test sequence and a reference sequence is the number of identical components which are shared by the two aligned sequences divided by the total number of components in the reference sequence segment, *i.e.,* the entire reference sequence or a smaller defined part of the reference sequence. Percent sequence identity is represented as the identity fraction times 100. The comparison of one or more polynucleotide sequences may be to a full-length polynucleotide sequence or a portion thereof, or to a longer polynucleotide sequence.

As used herein, the term "homology" refers to the level of similarity or percent identity between polynucleotide sequences in terms of percent nucleotide positional identity, *i*.*e*., sequence similarity or identity. As used herein, the term homology also refers to the concept of similar functional properties among different polynucleotide molecules, e.g., promoters that have similar function may have homologous cis-elements. Polynucleotide molecules are homologous when under certain conditions they specifically hybridize to form a duplex molecule. Under these conditions, referred to as stringency conditions, one polynucleotide molecule can be used as a probe or primer to identify other polynucleotide molecules that share homology. The phrase "stringent conditions" is functionally defined with regard to the hybridization of a nucleic-acid probe to a target nucleic acid (*i*.*e*., to a particular nucleic-acid sequence of interest) by the specific hybridization procedure discussed in Molecular Cloning: A Laboratory Manual, 3rd edition Volumes 1, 2, and 3. J.F. Sambrook, D.W. Russell, and N. Irwin, Cold Spring Harbor Laboratory Press, 2000 (referred to herein as Sambrook *et al*.). Accordingly, the nucleotide sequences of the invention may be used for their ability to selectively form duplex molecules with complementary stretches of polynucleotide molecule fragments. Depending on the application envisioned one would desire to employ varying conditions of hybridization to achieve varying degrees of selectivity of probe towards target sequence. For applications requiring high selectivity, one will typically desire to employ relatively high stringent conditions to form the hybrids, e.g., one will select relatively low salt and/or high temperature conditions, such as provided by about 0.02 M to about 0.15 M NaCl at temperatures of about 50°C to about 70°C. A high stringent condition, for example, is to wash the hybridization filter at least twice with high-stringency wash buffer (0.2X SSC, 0.1% SDS, 65°C). Appropriate moderate stringency conditions that promote DNA hybridization, for example, 6.0 x sodium chloride/sodium citrate (SSC) at about 45°C, followed by a wash of 2.0 x SSC at 50°C, are known to those skilled in the art. Additionally, the salt concentration in the wash step can be selected from a low stringency of about 2.0 x SSC at 50°C to a high stringency of about 0.2 x SSC at 50°C. Additionally, the temperature in the wash step can be increased from low stringency conditions at room temperature, about 22°C, to high stringency conditions at about 65°C. Both temperature and salt may be varied, or either the temperature or the salt concentration may be held constant while the other variable is changed. Such selective conditions tolerate little mismatch between the probe and the template or target strand. Detection of polynucleotide molecules via hybridization is well known to those of skill in the art, and the teachings of U.S. Patents 4,965,188 and 5,176,995 are exemplary of the methods of hybridization analyses.

Homology can also be determined by computer programs that align polynucleotide sequences and estimate the ability of polynucleotide molecules to form duplex molecules under certain stringency conditions. Polynucleotide molecules from different sources that share a high degree of homology are referred to as "homologues".

Methods well known to one skilled in the art may be used to identify promoters of interest having activity similar to the promoters described herein. For example, cDNA libraries may be constructed using cells or tissues of interest and screened to identify genes having an expression pattern similar to that of the promoters described herein. The cDNA sequence for the identified gene may then be used to isolate the gene's promoter for further characterization. See, for example U.S. Patents 6,096,950; 5,589,583; and 5,898,096, incorporated herein by reference. Alternately, transcriptional profiling or electronic northern techniques may be used to identify genes having an expression pattern similar to that of the promoters described herein. Once these genes have been identified, their promoters may be isolated for further characterization. See, for example U.S. Patents 6,506,565 and 6,448,387, incorporated herein by reference. The electronic northern technique refers to a computer-based sequence analysis which allows sequences from multiple cDNA libraries to be compared electronically based on parameters the researcher identifies including abundance in EST populations in multiple cDNA libraries, or exclusively to EST sets from one or combinations of libraries. The transcriptional profiling technique is a high-throughput method used for the systematic monitoring of gene expression profiles for thousands of genes. This DNA chip-based technology arrays thousands of cDNA sequences on a support surface. These arrays are simultaneously hybridized to a population of labeled cDNA probes prepared from RNA samples of different cell or tissue types, allowing direct comparative analysis of expression. This approach may be used for the isolation of regulatory sequences such as promoters associated with those genes.

In another embodiment, the promoter disclosed herein can be modified. Those skilled in the art can create promoters that have variations in the polynucleotide sequence. The polynucleotide sequences of the promoters of the present invention as shown in SEQ ID NOs: 1, 2, or 5 may be modified or altered to enhance their control characteristics. One preferred method of alteration of a polynucleotide sequence is to use PCR to modify selected nucleotides or regions of sequences. These methods are well known to those of skill in the art. Sequences can be modified, for example by insertion, deletion, or replacement of template sequences in a PCR-based DNA modification approach. A "variant" is a promoter containing changes in which one or more nucleotides of an original promoter is deleted, added, and/or substituted, preferably while substantially maintaining promoter function. For example, one or more base pairs may be deleted from the 5' or 3' end of a promoter to produce a "truncated" promoter. One or more base pairs can also be inserted, deleted, or substituted internally to a promoter. In the case of a promoter fragment, variant promoters can include changes affecting the transcription of a minimal promoter to which it is operably linked. A minimal or basal promoter is a polynucleotide molecule that is capable of recruiting and binding the basal transcription machinery. One example of basal transcription machinery in eukaryotic cells is the RNA polymerase II complex and its accessory proteins. Variant promoters can be produced, for example, by standard DNA mutagenesis techniques or by chemically synthesizing the variant promoter or a portion thereof.

Novel chimeric promoters can be designed or engineered by a number of methods. Many promoters contain cis-elements that activate, enhance, or define the strength and/or specificity of the promoter. For example promoters may contain "TATA" boxes defining the site of transcription initiation and other cis-elements located upstream of the transcription initiation site that modulate transcription levels. For example, a chimeric promoter may be produced by fusing a first promoter fragment containing the activator cis-element from one promoter to a second promoter fragment containing the activator cis-element from another promoter; the resultant chimeric promoter may cause an increase in expression of an operably linked transcribable polynucleotide molecule. Promoters can be constructed such that promoter fragments or elements are operably linked, for example, by placing such a fragment upstream of a minimal promoter. The cis-elements and fragments of the present invention can be used for the construction of such chimeric promoters. Methods for construction of chimeric and variant promoters of the present invention include, but are not limited to, combining control elements of different promoters or duplicating portions or regions of a promoter (*see*, for example, U.S. Patents 4,990,607; 5,110,732; and 5,097,025, all of which are herein incorporated by reference). Those of skill in the art are familiar with the standard resource materials that describe specific conditions and procedures for the construction, manipulation, and isolation of macromolecules (e.g., polynucleotide molecules, plasmids, etc.), as well as the generation of recombinant organisms and the screening and isolation of polynucleotide molecules.

In another embodiment, a promoter comprising the polynucleotide sequence shown in SEQ ID NOs: 1, 2, or 5 includes any length of said polynucleotide sequence that is capable of regulating an operably linked transcribable polynucleotide molecule. For example, the promoters as disclosed in SEQ ID NOs: 1, 2, or 5 may be truncated or portions deleted and still be capable of regulating transcription of an operably linked polynucleotide molecule. In a related embodiment, a cis-element of the disclosed promoters may confer a particular specificity such as conferring enhanced expression of operably linked polynucleotide molecules in certain tissues and therefore is also capable of regulating transcription of operably linked polynucleotide molecules. Consequently, any fragments, portions, or regions of the promoters comprising the polynucleotide sequence shown in SEQ ID NOs: 1, 2, or 5 can be used as regulatory polynucleotide molecules, including but not limited to cis-elements or motifs of the disclosed polynucleotide molecules. Substitutions, deletions, insertions, or any combination thereof can be combined to produce a final construct.

### Polynucleotide Constructs

As used herein, the term "construct" refers to any recombinant polynucleotide molecule such as a plasmid, cosmid, virus, autonomously replicating polynucleotide molecule, phage, or linear or circular single-stranded or double-stranded DNA or RNA polynucleotide molecule, derived from any source, capable of genomic integration or autonomous replication, comprising a polynucleotide molecule where one or more polynucleotide molecule has been linked in a functionally operative manner.

As used herein, the phrase "operably linked" refers to a first polynucleotide molecule, such as a promoter, connected with a second transcribable polynucleotide molecule, such as a gene of interest, where the polynucleotide molecules are so arranged that the first polynucleotide molecule affects the function of the second polynucleotide molecule. Preferably, the two polynucleotide molecules are part of a single contiguous polynucleotide molecule and more preferably are adjacent. For example, a promoter is operably linked to a gene of interest if the promoter regulates or mediates transcription of the gene of interest in a cell.

As used herein, the phrase "transcribable polynucleotide molecule" refers to any polynucleotide molecule capable of being transcribed into a RNA molecule. Methods are known for introducing constructs into a cell in such a manner that the transcribable polynucleotide molecule is transcribed into a functional mRNA molecule that is translated and therefore expressed as a protein product. Constructs may also be constructed to be capable of expressing antisense RNA molecules, in order to inhibit translation of a specific RNA molecule of interest. For the practice of the present invention, conventional compositions and methods for preparing and using constructs and host cells are well known to one skilled in the art (*see*, for example, Sambrook *et al*.).

Constructs of the present invention would typically contain a promoter operably linked to a transcribable polynucleotide molecule operably linked to a 3' transcription termination polynucleotide molecule. In addition, constructs may include but are not limited to additional regulatory polynucleotide molecules from the 3'-untranslated region (3' UTR) of plant genes (e.g., a 3' UTR to increase mRNA stability of the mRNA, such as the PI-II termination region of potato or the octopine or nopaline synthase 3' termination regions). Constructs may include but are not limited to the 5' untranslated regions (5' UTR) of an mRNA polynucleotide molecule which can play an important role in translation initiation and can also be a genetic component in a plant expression construct. For example, non-translated 5' leader polynucleotide molecules derived from heat shock protein genes have been demonstrated to enhance gene expression in plants (*see*, for example, U.S. Patents 5,659,122 and 5,362,865; and U.S. Published Application 2002/0192812, herein incorporated by reference). These additional upstream and downstream regulatory polynucleotide molecules may be derived from a source that is native or heterologous with respect to the other elements present on the promoter construct.

Thus, constructs of the present invention comprise promoters such as those provided in SEQ ID NOs: 1, 2, or 5 or modified as described above, operatively linked to a transcribable polynucleotide molecule so as to direct transcription of said transcribable polynucleotide molecule at a desired level or in a desired tissue or developmental pattern upon introduction of said construct into a plant cell. In some cases, the transcribable polynucleotide molecule comprises a protein-coding region of a gene, and the promoter provides for transcription of a functional mRNA molecule that is translated and expressed as a protein product. Constructs may also be constructed for transcription of antisense RNA molecules or other similar inhibitory RNA in order to inhibit expression of a specific RNA molecule of interest in a target host cell.

Exemplary transcribable polynucleotide molecules for incorporation into constructs of the present invention include, for example, DNA molecules or genes from a species other than the target gene species, or even genes that originate with or are present in the same species, but are incorporated into recipient cells by genetic engineering methods rather than classical reproduction or breeding techniques. Exogenous gene or genetic element is intended to refer to any gene or DNA molecule that is introduced into a recipient cell. The type of DNA included in the exogenous DNA can include DNA that is already present in the plant cell, DNA from another plant, DNA from a different organism, or a DNA generated externally, such as a DNA molecule containing an antisense message of a gene, or a DNA molecule encoding an artificial or modified version of a gene.

The promoters of the present invention can be incorporated into a construct using marker genes as described and tested in transient analyses that provide an indication of gene expression in stable plant systems. As used herein the phrase "marker gene" refers to any transcribable polynucleotide molecule whose expression can be screened for or scored in some way. Methods of testing for marker gene expression in transient assays are known to those of skill in the art. Transient expression of marker genes has been reported using a variety of plants, tissues, and DNA delivery systems. For example, types of transient analyses can include but are not limited to direct gene delivery via electroporation or particle bombardment of tissues in any transient plant assay using any plant species of interest. Such transient systems would include but are not limited to electroporation of protoplasts from a variety of tissue sources or particle bombardment of specific tissues of interest. The present invention encompasses the use of any transient expression system to evaluate promoters or promoter fragments operably linked to any transcribable polynucleotide molecules, including but not limited to selected reporter genes, marker genes, or genes of agronomic interest. Examples of plant tissues envisioned to test in transients via an appropriate delivery system would include but are not limited to leaf base tissues, callus, cotyledons, roots, endosperm, embryos, floral tissue, pollen, and epidermal tissue.

Any scorable or screenable marker gene can be used in a transient assay. Preferred marker genes for transient analyses of the promoters or promoter fragments of the present invention include a GUS gene (U.S. Patent 5,599,670, herein incorporated by reference) or a GFP gene (U.S. Patent 5,491,084, herein incorporated by reference). The constructs containing the promoters or promoter fragments operably linked to a marker gene are delivered to the tissues and the tissues are analyzed by the appropriate mechanism, depending on the marker. The quantitative or qualitative analyses are used as a tool to evaluate the potential expression profile of the promoters or promoter fragments when operatively linked to genes of agronomic interest in stable plants.

Thus, in one preferred embodiment, a polynucleotide molecule of the present invention as shown in SEQ ID NOs: 1, 2, or 5, or fragments, variants, or derivatives thereof is incorporated into a construct such that a promoter of the present invention is operably linked to a transcribable polynucleotide molecule that provides for a selectable, screenable, or scorable marker. Markers for use in the practice of the present invention include, but are not limited to transcribable polynucleotide molecules encoding β-glucuronidase (GUS), green fluorescent protein (GFP), luciferase (LUC), proteins that confer antibiotic resistance, or proteins that confer herbicide tolerance. Useful antibiotic resistance markers, including those encoding proteins conferring resistance to kanamycin (nptII), hygromycin B (aph IV), streptomycin or spectinomycin (aad, spec/strep), and gentamycin (aac3 and aacC4) are known in the art. Herbicides for which transgenic plant tolerance has been demonstrated and the method of the present invention can be applied, include but are not limited to: glyphosate, glufosinate, sulfonylureas, imidazolinones, bromoxynil, delapon, cyclohezanedione, protoporphyrionogen oxidase inhibitors, and isoxasflutole herbicides. Polynucleotide molecules encoding proteins involved in herbicide tolerance are known in the art, and include, but are not limited to a polynucleotide molecule encoding 5-enolpyruvylshikimate-3-phosphate synthase (EPSPS) described in U.S. Patents 5,627,061; 5,633,435; and 6,040,497; and *aroA* described in U.S. Patent 5,094,945 for glyphosate tolerance; a polynucleotide molecule encoding bromoxynil nitrilase (*Bxn*) described in U.S. Patent 4,810,648 for Bromoxynil tolerance; a polynucleotide molecule encoding phytoene desaturase (*crtI*) described in Misawa et al., Plant J., 4:833-840 (1993) and Misawa et al., Plant J., 6:481-489 (1994) for norflurazon tolerance; a polynucleotide molecule encoding acetohydroxyacid synthase (AHAS, *aka* ALS) described in Sathasiivan et al., Nucl. Acids Res., 18:2188-2193 (1990) for tolerance to sulfonylurea herbicides; and the *bar* gene described in DeBlock, et al., EMBO J., 6:2513-2519 (1987) for glufosinate and bialaphos tolerance.

In one preferred embodiment, a polynucleotide molecule of the present invention as shown in SEQ ID NOs: 1, 2, or 5, or fragments, variants, or derivatives thereof is incorporated into a construct such that a promoter of the present invention is operably linked to a transcribable polynucleotide molecule that is a gene of agronomic interest. As used herein, the phrase "gene of agronomic interest" refers to a transcribable polynucleotide molecule that includes but is not limited to a gene that provides a desirable characteristic associated with plant morphology, physiology, growth and development, yield, nutritional enhancement, disease or pest resistance, or environmental or chemical tolerance. The expression of a gene of agronomic interest is desirable in order to confer an agronomically important trait. A gene of agronomic interest that provides a beneficial agronomic trait to crop plants may be, for example, including, but not limited to genetic elements comprising herbicide resistance (U.S. Patents 5,633,435 and 5,463,175), increased yield (U.S. Patent 5,716,837), insect control (U.S. Patents 6,063,597; 6,063,756; 6,093,695; 5,942,664; and 6,110,464), fungal disease resistance (U.S. Patents 5,516,671; 5,773,696; 6,121,436; 6,316,407; and 6,506,962), virus resistance (U.S. Patents 5,304,730 and 6,013,864), nematode resistance (U.S. Patent 6,228,992), bacterial disease resistance (U.S. Patent 5,516,671), starch production (U.S. Patents 5,750,876 and 6,476,295), modified oils production (U.S. Patent 6,444,876), high oil production (U.S. Patents 5,608,149 and 6,476,295), modified fatty acid content (U.S. Patent 6,537,750), high protein production (U.S. Patent 6,380,466), fruit ripening (U.S. Patent 5,512,466), enhanced animal and human nutrition (U.S. Patents 5,985,605 and 6,171,640), biopolymers (U.S. Patent 5,958,745 and U.S. Published Application 2003/0028917), environmental stress resistance (U.S. Patent 6,072,103), pharmaceutical peptides (U.S. Patent 6,080,560), improved processing traits (U.S. Patent 6,476,295), improved digestibility (U.S. Patent 6,531,648) low raffinose (U.S. Patent 6,166,292), industrial enzyme production (U.S. Patent 5,543,576), improved flavor (U.S. Patent 6,011,199), nitrogen fixation (U.S. Patent 5,229,114), hybrid seed production (U.S. Patent 5,689,041), and biofuel production (U.S. Patent 5,998,700), the genetic elements and transgenes described in the patents listed above are herein incorporated by reference.

Alternatively, a transcribable polynucleotide molecule can effect the above mentioned phenotypes by encoding a non-translatable RNA molecule that causes the targeted inhibition of expression of an endogenous gene, for example via antisense, RNAi, or cosuppression-mediated mechanisms. The RNA could also be a catalytic RNA molecule (*i*.*e*., a ribozyme) engineered to cleave a desired endogenous mRNA product. Thus, any polynucleotide molecule that encodes a protein or mRNA that expresses a phenotype or morphology change of interest is useful for the practice of the present invention.

The constructs of the present invention are generally double Ti plasmid border DNA constructs that have the right border (RB or AGRtu.RB) and left border (LB or AGRtu.LB) regions of the Ti plasmid isolated from *Agrobacterium tumefaciens* comprising a T-DNA, that along with transfer molecules provided by the *Agrobacterium* cells, permits the integration of the T-DNA into the genome of a plant cell. The constructs also contain the plasmid backbone DNA segments that provide replication function and antibiotic selection in bacterial cells, for example, an *E*. *coli* origin of replication such as *ori*322, a broad host range origin of replication such as *ori*V or *ori*Ri, and a coding region for a selectable marker such as Spec/Strp that encodes for Tn7 aminoglycoside adenyltransferase (*aadA*) conferring resistance to spectinomycin or streptomycin, or a gentamicin (Gm, Gent) selectable marker gene. For plant transformation, the host bacterial strain is often *Agrobacterium tumefaciens* ABI, C58, or LBA4404, however, other strains known to those skilled in the art of plant transformation can function in the present invention.

### Transformed Plants And Plant Cells

As used herein, the term "transformed" refers to a cell, tissue, organ, or organism into which has been introduced a foreign polynucleotide molecule, such as a construct. Preferably, the introduced polynucleotide molecule is integrated into the genomic DNA of the recipient cell, tissue, organ, or organism such that the introduced polynucleotide molecule is inherited by subsequent progeny. A "transgenic" or "transformed" cell or organism also includes progeny of the cell or organism and progeny produced from a breeding program employing such a transgenic plant as a parent in a cross and exhibiting an altered phenotype resulting from the presence of a foreign polynucleotide molecule. A plant transformation construct containing a promoter of the present invention may be introduced into plants by any plant transformation method. Methods and materials for transforming plants by introducing a plant expression construct into a plant genome in the practice of this invention can include any of the well-known and demonstrated methods including electroporation as illustrated in U.S. Patent 5,384,253; microprojectile bombardment as illustrated in U.S. Patents 5,015,580; 5,550,318; 5,538,880; 6,160,208; 6,399,861; and 6,403,865; *Agrobacterium*-mediated transformation as illustrated in U.S. Patents 5,635,055; 5,824,877; 5,591,616; 5,981,840; and 6,384,301; and protoplast transformation as illustrated in U.S. Patent 5,508,184, all of which are incorporated herein by reference.

Methods for specifically transforming dicots are well known to those skilled in the art. Transformation and plant regeneration using these methods have been described for a number of crops including, but not limited to, cotton (*Gossypium hirsutum*), soybean (*Glycine max*), peanut (*Arachis hypogaea*), alfalfa (*Medicago sativa*), and members of the genus *Brassica.* It is apparent to those of skill in the art that a number of transformation methodologies can be used and modified for production of stable transgenic plants from any number of dicotyledonous target crops of interest.

Methods for specifically transforming monocots are well known to those skilled in the art. Transformation and plant regeneration using these methods have been described for a number of crops including, but not limited to, barley (*Hordeum vulgarae*); maize (*Zea mays*); oats (*Avena sativa*); orchard grass (*Dactylis glomerata*); rice (*Oryza sativa*, including indica and japonica varieties); sorghum (*sorghum bicolor*); sugar cane (*Saccharum sp*);tall fescue (*Festuca arundinacea*); turfgrass (*Agrostis*); and wheat (*Triticum aestivum*). It is apparent to those of skill in the art that a number of transformation methodologies can be used and modified for production of stable transgenic plants from any number of monocotyledonous target crops of interest.

Many seeds, nuts, and kernels contain oil that can be extracted and used in cooking, as an ingredient in other foods, as a nutritional supplement, as a raw material for the manufacture of soap, body and hair oils, detergents, paints, as well as, replacements for certain petroleum-based lubricants and fuels. As used herein, these seeds, nuts, and kernels collectively are termed "oil seeds" (National Sustainable Agriculture Information Service (ATTRA), Fayetteville, AR). Table 1 lists seeds, nuts, and kernels commonly classified as oil seeds.

**Table 1. Oil containing seeds, nuts, kernels**

| | | |
|---|---|---|
| Apricot stones | Black currant | Red pepper |
| Avocado | Jojoba | Brazil nut |
| Cotton seed | Coffee | Passion fruit |
| Billberry | Cocoa | Pecan |
| Borage | Coriander | Pistachio |
| Stinging nettle | Caraway seed | Rape seed |
| Beech nut | Pumpkin seed | Castor bean |
| Calendula | Linseed | Sea buckthorn |
| Cashew nut | Mace | Mustard seed |
| Copra (dried coconut) | Corn seed | Sesame seed |
| Safflower | Macadamia nut | Soybean |
| Groundnut | Almonds | Sunflower seed |
| Spurge | Melon seed | Tropho plant |
| Rubber seed | Poppy | Tomato seed |
| Rose hip | Nutmeg | Grape seed |
| Hemp | Evening primrose | Walnut |
| Hazelnut | Neem seed | Citrus seed |
| Raspberry | Niger seed | Canola |
| Elderberry | Palm kernel | |

In another embodiment, the invention provides a method of making a vegetable oil, comprising the steps of incorporating into the genome of an oilseed plant a promoter of the present invention operably linked to a transcribable polynucleotide molecule conferring altered oil and/or protein content, growing the oilseed plant to produce oilseeds, and extracting the oil and/or protein from the oilseed.

The transformed plants are analyzed for the presence of the genes of interest and the expression level and/or profile conferred by the promoters of the present invention. Those of skill in the art are aware of the numerous methods available for the analysis of transformed plants. For example, methods for plant analysis include, but are not limited to Southern blots or northern blots, PCR-based approaches, biochemical analyses, phenotypic screening methods, field evaluations, and immunodiagnostic assays.

The seeds of this invention can be harvested from fertile transgenic plants and be used to grow progeny generations of transformed plants of this invention including hybrid plant lines comprising the construct of this invention and expressing a gene of agronomic interest.

The promoter of the present invention provides for differential expression in plant tissues, preferably in at least one plant seed tissue that includes seed coat, embryo, aleurone, and endosperm. The promoters are herein referred to as "seed enhanced promoters."

The phrase "micronutrient content" means the amount of micronutrients, *i.e.,* vitamins A, E, K, tocopherols, tocotrienols, or carotenoids, within a seed expressed on a per weight basis.

The phrase "oil content" means oil level, which may be determined, for example, by low-resolution ¹H nuclear magnetic resonance (NMR) (Tiwari et al.,JAOCS,51:104-109 (1974) or Rubel, JAOCS,71:1057-1062 (1994)) or near infrared transmittance (NIT) spectroscopy (Orman et al., JAOCS, 69(10):1036-1038 (1992); Patrick et al.,JAOCS,74(3):273-276 (1997)).

The phrase "protein quality" means the level of one or more essential amino acids, whether free or incorporated in protein, namely histidine, isoleucine, leucine, lysine, methionine, cysteine, phenylalanine, tyrosine, threonine, tryptophan, and valine.

As used herein, the phrase "oil composition" means the ratio of different fatty acid or oil components within a sample. Such a sample may be a plant or plant part, such as a seed. Such a sample may also be a collection of plant parts.

As used herein, the phrase "percentage content" in a preferred embodiment means the percent by total weight of a particular component, relative to other similar of related components.

As used herein, the phrase "enhanced oil" or "oil enhancing" includes increased oil yield or altered oil composition.

The following examples are included to demonstrate preferred embodiments of the invention. It should be appreciated by those of skill in the art that the techniques disclosed in the examples that follow represent techniques discovered by the inventors to function well in the practice of the invention. However, those of skill in the art should, in light of the present disclosure, appreciate that many changes can be made in the specific embodiments that are disclosed and still obtain a like or similar result without departing from the spirit and scope of the invention, therefore all matter set forth or shown in the accompanying drawings is to be interpreted as illustrative and not in a limiting sense.

### EXAMPLES

### Example 1: Isolation of the Promoters P-Gm.701202739 and P-Gm.701209813

ESTs enhanced for seed-coat expression were identified by library subtraction and electronic Northerns using the PhytoSeq database SOYMON035. DNA from the soybean cultivar A3244 was extracted and used to construct a GenomeWalker (BD Biosciences, Palo Alto, CA) library. The two promoters from the ESTs identified above were isolated by PCR amplification from the GenomeWalker libraries: 701202739H1 (0.76 kb) and 701209813H1 (0.78 kb).

The 5'-upstream regulatory sequences for the putative soybean seed-coat promoters were cloned and the sequences were verified: P.701202739H1 (SEQ ID NO: 1) (pMON57315) and 701209813H1 (SEQ ID NO: 2) (pMON57314).

### Example 2: Isolation of the Promoter P-At.TT2

The *Arabidopsis thaliana* TT2 gene encodes an R2R3 MYB domain protein involved in proanthocyanidin accumulation in developing seed coats. A promoter sequence was identified by first BLASTing the coding sequence (AJ299452) against a Monsanto *Arabidopsis thaliana* sequence database ArabGDNA which contains all *Arabidopsis* genomic sequences from all sources. The P1 Clone MOK9 (AB015477) contained the TT2 coding sequence (basepairs 64541-67240). Primers were designed to amplify the sequences upstream of the TT2 coding region (corresponding to basepairs 64658-65620 of P1 MOK9) from *Arabidopsis* genomic DNA (Strain ID CS3176). The predicted ATG of TT2 was converted to an NcoI site and an SrfI site was added to the 5' end of the second primer to facilitate cloning. Primer sequences were SEQ ID NO: 3 and SEQ ID NO: 4. The promoter sequence (SEQ ID NO: 5) was cloned into PCR2.1 Topo (Invitrogen, Carlsbad, CA) to give pMON65418.

### Example 3: Constructs for Arabidopsis Transformation

### pMON65410 (P-Gm.701202739-0:3:1::GUS)

A 698 bp fragment containing P-Gm.701202739-0:3:1 was removed from pMON57315 by digestion with PstI and NcoI. The fragment was ligated into pMON69802, which had been digested with Sse8387I and NcoI. The resulting plasmid, containing P-Gm.701202739-0:3:11 driving the *E. coli uid*A gene and with the napin 3' UTR was named pMON65410. The nucleic acid sequence was determined using known methodology and the integrity of the cloning junctions confirmed. This vector was used in the subsequent transformation of *Arabidopsis*.

### pMON65409 (P-Gm.701209813-0:3:1::GUS)

A 704 bp fragment containing P-Gm.701209813-0:3:1 was removed from pMON57314 by digestion with PstI and NcoI. The fragment was ligated into pMON69802, which had been digested with Sse8387I and NcoI. The resulting plasmid, containing P-Gm.701209813-0:3:11 driving the *E. coli uid*A gene and with the napin 3' UTR was named pMON65409. The nucleic acid sequence was determined using known methodology and the integrity of the cloning junctions confirmed. This vector was used in the subsequent transformation of *Arabidopsis*.

Transgenic *Arabidopsis thaliana* plants were obtained as described by Bent et al., Science, 265:1856-1860 (1994) or Bechtold et al., C.R.Acad.Sci, Life Sciences, 316:1194-1199 (1993). Cultures of *Agrobacterium tumefaciens* strain ABI containing either of the transformation vectors pMON65410 or pMON65409 were grown overnight in LB (10% bacto-tryptone, 5% yeast extract, and 10% NaCl with kanamycin (75 mg/L), chloramphenicol (25 mg/L), and spectinomycin (100 mg/L)). The bacterial culture was centrifuged and resuspended in 5% sucrose + .05% Silwet-77 solution. The aerial portions of whole *Arabidopsis thaliana* plants (at about 5-7 weeks of age) were immersed in the resulting solution for 2-3 seconds. The excess solution was removed by blotting the plants on paper towels. The dipped plants were placed on their side in a covered flat and transferred to a growth chamber at 19°C. After 16 to 24 hours the dome was removed and the plants were set upright. When plants had reached maturity, water was withheld for 2-7 days prior to seed harvest. Harvested seed was passed through a stainless steel mesh screen (40 holes/inch) to remove debris. The harvested seed was stored in paper coin envelopes at room temperature until analysis.

*Arabidopsis* seeds were surfaced sterilized using a vapor phase sterilization protocol. An open container of seeds was placed in a dessicator with a beaker containing 100 ml of household bleach. Immediately prior to sealing the dessicator, 3 ml concentrated HCl was added to the bleach. The dessicator was sealed and a vacuum was applied to allow sterilization by chlorine fumes. Seeds were incubated for several hours. Sterilized seed were sprinkled onto *Arabidopsis* Germination Media (MS Salts (1X); sucrose (1%); myo-Inositol (100 mg/L); Thiamine-HCl (1 mg/L); Pyridoxine-HCl (500 mg/L); Nicotinic Acid (500 mg/L); MES pH 5.7 (0.05%); and Phytagar (0.7%)) supplemented with 50 mg/L glyphosate.

### Example 4: Promoter Characterization in Transgenic Arabidopsis Plants

Expression of β-glucuronidase (the product of the *E. coli uidA* gene) was analyzed in *Arabidopsis thaliana* plants transformed with pMON65409 or pMON65410 using histochemical staining. Up to 16 glyphosate resistant seedlings were transplanted to 2¼-inch pots, one seedling per pot, containing MetroMix 200 and were grown under the conditions described above until the initial siliques that had formed began to desiccate. Tissue (rosette leaf, cauline leaf, stem, flowers, floral buds, and developing siliques) was removed from each T1 plant for subsequent histochemical staining. Tissue (rosette leaf, cauline leaf, stem, flowers, floral buds, and developing siliques) was removed from each T1 plant for subsequent histochemical staining.

Tissues, prepared as described above, were incubated for approximately 24 hours at 37°C in a solution containing 50 mM NaPO₄ (pH 7.2); 100 µM potassium ferricyanide; 100 µM potassium ferrocyanide, 0.03% Triton X-100; 20% methanol and 2.5 mg/ml 5-bromo-4-chloro-3-indoyl glucuronic acid (X-gluc). The stained tissue was cleared of chlorophyll by an overnight incubation in 70% ethanol/30% H₂O at 37°C. Stained tissues were photographed immediately or transferred to a solution of 70% ethanol/30% glycerol (v/v) and stored at 4°C until photographed.

For pMON65409, 5 out of 5 plants screened had detectable levels of activity in seed from at least one time point. For pMON65410, 5 out of 5 plants screened had detectable levels of activity in seed from at least one time point.

### Example 5: Constructs for Canola Transformation

### pMON65431 (P-Gm.701202739-0:3:1::GUS)

A 745 bp fragment containing P-Gm.701202739-0:3:1 was removed from PMON57315 by digestion with NotI and NcoI. The fragment was ligated into pMON65424, which had been digested with NotI and NcoI. The resulting plasmid, containing P-Gm.701202739-0:3:11 driving the *E. coli uid*A gene and with the napin 3' UTR was named pMON65431. The nucleic acid sequence was determined using known methodology and confirmed the integrity of the cloning junctions. This vector was used in the subsequent transformation of Canola.

### pMON65432 (P-Gm.701209813-0:3:1::GUS)

A 746 bp fragment containing P-Gm.701209813-0:3:1 was removed from pMON57314 by digestion with NotI and NcoI. The fragment was ligated into pMON65424, which had been digested with NotI and NcoI. The resulting plasmid, containing P-Gm.701209813-0:3:11 driving the *E. coli uid*A gene and with the napin 3' UTR was named pMON65432. The nucleic acid sequence was determined using known methodology and confirmed the integrity of the cloning junctions. This vector was used in the subsequent transformation of Canola.

### pMON65427 (P-At.TT2-0:3:2::GUS)

A 966 bp fragment containing P-At.TT2-0:3:2 was removed from pMON65418 by digestion with SmaI and NcoI. The fragment was ligated into pMON65424, which had been digested with PmeI and NcoI. The resulting plasmid, containing P-At.TT2-0:3:21 driving the *E*. *coli uid*A gene and with the napin 3' UTR was named pMON65427. The nucleic acid sequence was determined using known methodology and confirmed the integrity of the cloning junctions. This vector was used in the subsequent transformation of Canola.

The vectors pMON65422, 65431 and pMON65432 were introduced into *A*. *tumefaciens* strain ABI for transformation into *Brassica napus.* Canola plants were transformed using the protocol described by Moloney and Radke in U.S. Patent 5,720,871. Briefly, seeds of *B*. *napus* cv Ebony were planted in 2-inch pots containing Metro Mix 350 (The Scotts Company, Columbus, OH). The plants were grown in a growth chamber at 24°C, and a 16/8 hour photoperiod, with light intensity of 400 µEm⁻² sec⁻¹ (HID lamps). After 2-1/2 weeks, the plants were transplanted into 6-inch pots and grown in a growth chamber at 15/10°C day/night temperature, 16/8 hour photoperiod, light intensity of 800 µEm⁻² sec⁻¹ (HID lamps).

Four terminal internodes from plants just prior to bolting or in the process of bolting but before flowering were removed and surface sterilized in 70% v/v ethanol for 1 minute, 2% w/v sodium hypochlorite for 20 minutes and rinsing 3 times with sterile deionized water. Six to seven stem segments were cut into 5 mm discs, maintaining orientation of basal end.

The *Agrobacterium* culture used to transform Canola was grown overnight on a rotator shaker at 24°C in 2 mls of Luria Broth, LB (10% bacto-tryptone, 5% yeast extract, and 10% NaCl) containing 50 mg/l kanamycin, 24 mg/l chloramphenicol, and 100 mg/l spectinomycin. A 1:10 dilution was made in MS media (Murashige and Skoog, Physiol.Plant., 15:473-497, 1962) giving approximately 9x10⁸ cells per ml. The stem discs (explants) were inoculated with 1.0 ml of *Agrobacterium* and the excess was aspirated from the explants. The explants were placed basal side down in petri plates containing media comprising 1/10 MS salts, B5 vitamins (1% inositol; 0.1% thiamine HCl; 0.01% nicotinic acid; 0.01% pyridoxine-HCl), 3% sucrose, 0.8% agar, pH 5.7, 1.0 mg/l 6-benzyladenine (BA). The plates were layered with 1.5 ml of media containing MS salts, B5 vitamins, 3% sucrose, pH 5.7, 4.0 mg/l p-chlorophenoxyacetic acid, 0.005 mg/l kinetin and covered with sterile filter paper.

Following a 2 to 3 day co-culture, the explants are transferred to deep dish petri plates containing MS salts, B5 vitamins, 3% sucrose, 0.8% agar, pH 5.7, 1 mg/l BA, 500 mg/l carbenicillin, 50mg/l cefotaxime, 200 mg/l kanamycin, or 175 mg/l gentamycin for selection. Seven explants were placed on each plate. After 3 weeks they were transferred to fresh media, 5 explants per plate. The explants were cultured in a growth room at 25°C, continuous light (Cool White).

The transformed plants were grown in a growth chamber at 22°C in a 16-8 hours light-dark cycle with light intensity of 220 µEm⁻²s⁻¹ for several weeks before transferring to the greenhouse. Plants were maintained in a greenhouse under standard conditions. Developing seed was harvested at various stages after pollination and stored at minus 70°C. Mature seed was collected and stored under controlled conditions consisting of about 17°C and 30% humidity.

### Example 6: Promoter Characterization in Transgenic Canola Plants

Up to 5 siliques were harvested from individual R0 plants transformed with pMON65427, pMON65431, or pMON65432 at several time points after pollination. Siliques were scored with an 18 gauge needle to allow the staining solution to contact the developing seed. The siliques were incubated for approximately 24 hours at 37°C in a solution containing 50 mM NaPO₄ (pH 7.2); 100 µM potassium ferricyanide; 100 µM potassium ferrocyanide, 0.03% Triton X-100; 20% methanol and 2.5 mg/ml 5-bromo-4-chloro-3-indoyl glucuronic acid (X-gluc). The stained tissue was cleared of chlorophyll by an overnight incubation in 70% ethanol/30% H₂O at 37°C. Stained tissues were photographed immediately or transferred to a solution of 70% ethanol/30% glycerol (v/v) and stored at 4°C until photographed. Samples were scored positive (+) or negative (-) for blue color.

For pMON65431, 6 out of 10 plants screened have detectable levels of activity in seed from at least one time point. Time course data for individual transformants is presented in Tables 2 and 3.

**Table 2. P-Gm.701202739-0:3:1 Expression in Developing Canola Seed**

| | | *Days After Pollination* | | | | | | | | | |
|---|---|---|---|---|---|---|---|---|---|---|---|
| *Construct* | *Event* | *3* | *6* | *9* | *12* | *15* | *20* | *25* | *30* | *35* | *40* |
| pMON65431 | BN_G2244 | - | - | - | - | - | - | - | - | - | - |
| pMON65431 | BN_G2245 | - | - | - | - | - | - | - | - | - | - |
| pMON65431 | BN_G2246 | + | + | + | + | + | - | - | - | - | - |
| pMON65431 | BN_G2308 | - | - | - | - | - | - | - | - | - | - |
| pMON65431 | BN_G2309 | - | - | - | - | - | - | - | - | - | - |
| pMON65431 | BN_G2310 | + | + | + | + | + | + | + | - | - | - |
| pMON65431 | BN_G2311 | - | + | + | + | + | + | - | - | - | - |
| pMON65431 | BN_G2312 | + | - | + | - | + | + | - | - | - | - |
| pMON65431 | BN_G2352 | - | + | + | + | + | - | - | - | - | - |
| pMON65431 | BN_G2353 | + | + | + | + | + | + | - | - | - | - |
| Control | SP30052 | - | - | - | - | - | - | - | - | - | - |

**Table 3. P-Gm.701209813-0:3:1 Expression in Developing Canola Seed**

| | | *Days After Pollination* | | | | | | | | | |
|---|---|---|---|---|---|---|---|---|---|---|---|
| *Construct* | *Event* # | *3* | *6* | *9* | *12* | *15* | *20* | *25* | *30* | *35* | *40* |
| pMON65432 | BN_G2064 | - | - | - | - | - | - | - | - | - | - |
| pMON65432 | BN_G2065 | - | - | - | - | - | - | - | - | - | - |
| pMON65432 | BN_G2152 | - | - | - | - | - | + | - | - | - | - |
| pMON65432 | BN_G2153 | - | - | - | - | - | - | - | - | - | - |
| pMON65432 | BN_G2154 | - | - | - | - | - | - | - | - | - | - |
| pMON65432 | BN_G2155 | - | - | - | - | - | - | - | - | - | - |
| pMON65432 | BN_G2156 | - | - | - | - | - | - | - | - | - | - |
| pMON65432 | BN_G2157 | - | - | - | - | - | - | - | - | - | - |
| pMON65432 | BN_G2158 | - | - | - | - | - | - | - | - | + | - |
| pMON65432 | BN_G2160 | - | - | - | - | - | - | - | - | - | - |
| Control | SP30052 | - | - | - | - | - | - | - | - | - | - |

For pMON65427, 10 out of 10 plants screened have detectable levels of activity in seed from at least one time point. Time course data for individual transformants is presented in Table 4.

**Table 4. P-At.TT2-0:3:2 Expression in Developing Canola Seed**

| | *Days After Pollination* | | | | | | | | | |
|---|---|---|---|---|---|---|---|---|---|---|
| *Construct* | *3* | *6* | *9* | *12* | *15* | *20* | *25* | *30* | *35* | *40* |
| pMON65427 | - | + | + | + | + | + | + | + | + | + |
| pMON65427 | - | + | + | + | + | + | + | + | + | + |
| pMON65427 | - | - | - | - | - | - | + | + | - | - |
| pMON65427 | - | - | - | - | - | - | - | - | + | - |
| pMON65427 | - | - | - | - | - | - | - | + | + | + |
| pMON65427 | - | - | + | + | - | + | + | + | + | + |
| pMON65427 | + | | + | + | + | + | + | + | + | + |
| pMON65427 | - | + | + | + | + | + | + | + | + | + |
| pMON65427 | | - | - | + | - | + | + | + | + | + |
| pMON65427 | - | | + | + | + | + | + | + | + | + |
| Control | - | - | - | - | - | - | - | - | - | - |

Having illustrated and described the principles of the present invention, it should be apparent to persons skilled in the art that the invention can be modified in arrangement and detail without departing from such principles. We claim all modifications that are within the spirit and scope of the appended claims. All publications and published patent documents cited in this specification are incorporated herein by reference to the same extent as if each individual publication or patent application is specifically and individually indicated to be incorporated by reference.

A promoter comprising a polynucleotide sequence selected from the group consisting of:
(a) a polynucleotide sequence comprising the sequence of SEQ ID NO:1, SEQ ID NO:2, or SEQ ID NO:5,
(b) a polynucleotide sequence comprising a fragment of the polynucleotide sequence of (a) capable of regulating transcription of an operably linked transcribable polynucleotide molecule; and
(c) a polynucleotide sequence comprising at least 70% sequence identity to the polynucleotide sequence of (a) or (b) capable of regulating transcription of an operably linked transcribable polynucleotide molecule.

The promoter as defined above, wherein said promoter comprises a polynucleotide sequence with from about 90% identity to about 99% sequence identity to the polynucleotide sequence of (a) or fragment of (b).

The promoter as defined above, wherein said promoter comprises a polynucleotide sequence with from about 80% identity to about 89% sequence identity to the polynucleotide sequence of (a) or fragment of (b).

The promoter as defined above, wherein said promoter comprises a polynucleotide sequence with from about 70% identity to about 79% sequence identity to the polynucleotide sequence of (a) or fragment of (b).

A construct comprising the promoter of claim 1 operably linked to a transcribable polynucleotide molecule.

The construct as defined above, wherein said transcribable polynucleotide molecule is a gene of agronomic interest.

The construct as defined above, wherein said transcribable polynucleotide molecule is a marker gene.

A transgenic plant or a part thereof stably transformed with the construct as defined above.

The transgenic plant as defined aobve, wherein said plant is a dicotyledonous plant selected from the group consisting of tobacco, tomato, potato, soybean, cotton, canola, sunflower, and alfalfa. The transgenic plant as defined above, wherein said transcribable polynucleotide molecule confers altered oil content in the seed to said transgenic plant.

The transgenic plant as defined above, wherein said transcribable polynucleotide molecule confers altered protein quality in the seed to said transgenic plant.

The transgenic plant as defined above, wherein said transcribable polynucleotide molecule confers altered micronutrient content to said transgenic plant.

A transgenic seed transformed with the construct as defined above.

A transgenic cell transformed with the construct as defined above.

Oil from the transgenic seed as defined above, wherein the oil comprises a detectable nucleic acid comprising the promoter as defined above.

Meal from the transgenic seed as defined above, wherein the meal comprises a detectable nucleic acid comprising the promoter as defined above.

A method of making a vegetable oil, comprising the steps of:
a) obtaining the seed as defined above; and
b) extracting oil from the seed.

A method of making a meal, comprising the steps of:
a) obtaining the plant or part thereof as defined above; and
b) preparing meal from the plant or part thereof.

A method of making food or feed comprising the steps of:
a) obtaining the plant or part thereof as defined above; and
b) preparing food or feed from the plant or part thereof.

## Claims

1. A promoter comprising a polynucleotide sequence selected from the group consisting of:
(a) a polynucleotide sequence comprising the sequence of SEQ ID NO:5,
(b) a polynucleotide sequence comprising a fragment of the polynucleotide sequence of (a) capable of regulating transcription of an operably linked transcribable polynucleotide molecule; and
(c) a polynucleotide sequence comprising at least 70% sequence identity to the polynucleotide sequence of (a) or (b) capable of regulating transcription of an operably linked transcribable polynucleotide molecule.

2. The promoter of claim 1, wherein said promoter comprises
(i) a polynucleotide sequence with from about 90% identity to about 99% sequence identity to the polynucleotide sequence of (a) or fragment of (b); or
(ii) a polynucleotide sequence with from about 80% identity to about 89% sequence identity to the polynucleotide sequence of (a) or fragment of (b); or
(iii) a polynucleotide sequence with from about 70% identity to about 79% sequence identity to the polynucleotide sequence of (a) or fragment of (b).

3. A construct comprising the promoter of claim 1 or 2 operably linked to a transcribable polynucleotide molecule.

4. The construct of claim 3, wherein
(i) said transcribable polynucleotide molecule is a gene of agronomic interest; or
(ii) said transcribable polynucleotide molecule is a marker gene.

5. A transgenic plant or a part thereof stably transformed with the construct of claim 3 or 4.

6. The transgenic plant of claim 5, wherein
(i) said plant is a dicotyledonous plant selected from the group consisting of tobacco, tomato, potato, soybean, cotton, canola, sunflower, and alfalfa; or
(ii) said transcribable polynucleotide molecule confers altered oil content in the seed to said transgenic plant; or
(iii) said transcribable polynucleotide molecule confers altered protein quality in the seed to said transgenic plant; or
(iv) said transcribable polynucleotide molecule confers altered micronutrient content to said transgenic plant.

7. A transgenic seed or cell transformed with the construct of claim 3 or 4.

8. Oil from the transgenic seed of claim 7, wherein the oil comprises a detectable nucleic acid comprising the promoter of claim 1.

9. Meal from the transgenic seed of claim 7, wherein the meal comprises a detectable nucleic acid comprising the promoter of claim 1.

10. A method of making a vegetable oil, comprising the steps of:
a) obtaining the seed of claim 7; and
b) extracting oil from the seed.

11. A method of making a meal, comprising the steps of:
a) obtaining the plant or part thereof of claim 5; and
b) preparing meal from the plant or part thereof.

12. A method of making food or feed comprising the steps of:
a) obtaining the plant or part thereof of claim 5; and
b) preparing food or feed from the plant or part thereof.
